# EUROPÄISCHE PATENTSCHRIFT

(11) **EP 4 472 530 B1**
(45) Veröffentlichungstag und Bekanntmachung des Hinweises auf die Patenterteilung: **01.04.2026**
(21) Anmeldenummer: 23705473.9
(22) Anmeldetag: 03.02.2023
(51) Int. Cl.: A61B 17/24, A46B 15/00, A61B 13/00

(54) **ZUNGENREINIGER**
TONGUE CLEANER
DISPOSITIF DE NETTOYAGE DE LANGUE

(30) Priorität: 05.02.2022 DE 102022102726
(43) Veröffentlichungstag der Anmeldung: 11.12.2024
(73) Patentinhaber: Stelzer, Richard, 83471 Schönau am Königssee (DE)
(72) Erfinder: Stelzer, Richard, 83471 Schönau am Königssee (DE)
(74) Vertreter: Naessens, Stephan
(86) Internationale Anmeldenummer: PCT/DE2023/100090
(87) Internationale Veröffentlichungsnummer: WO 2023/147824

(56) Entgegenhaltungen:
- CN-U- 205 286 575
- CN-U- 209 173 037
- DE-U1- 202016 001 011
- US-A1- 2009 111 069

## Beschreibung

Die vorliegende Erfindung betrifft einen Zungenreiniger zum Reinigen einer Zunge eines Patienten bzw. einer Person mit zumindest einem Grundkörper und mit einem Griffabschnitt, wobei dem Grundkörper zumindest ein Reinigungsbereich zum Reinigen und Absaugen von Anhaftungen auf der Zunge zugeordnet ist.

Beispielsweise aus der Druckschrift DE 20 2016 001 011 U1 ist ein Zungenreinigerkopf mit einem scheibenförmigen Körper zur Reinigung einer Zunge eines Patienten bekannt. Der scheibenförmige Körper weist eine nutzungsseitige scheibenförmige Vorderfläche auf. An der Vorderfläche ist eine Profilierung zum Abschaben von oberflächlich an der menschlichen Zunge anhaftenden Verschmutzungen ausgeformt. Die Profilierung wird durch Rippen gebildet. Der Körper weist ferner einen Schlauchanschluss auf, der über mehrere Kanäle mit der Profilierung kommuniziert, sodass über einen an den Schlauchanschluss angeschlossenen Ablaufschlauch gelöste Verschmutzungen per Unterdruck bzw. Vakuum abgesaugt werden.

Aus der Druckschrift US 2009/111069 A1 ist ein Zungenschaber zur Verwendung in Kombination mit einer Saugvorrichtung bekannt. Der Zungenschaber ist stabförmig ausgeführt. An dessen einem Ende ein Absaugschlauch befestigt ist und an dessen anderem Ende eine Absaugöffnung vorgesehen ist.

Aus den Druckschriften CN 205 286 575 U und CN 209 173 037 U ist jeweils ein Mundreiniger mit Saugfunktion bekannt, der einen Griff aufweist, an dem ein Reinigungskopf vorgesehen ist.

Es hat sich gezeigt, dass nicht nur bei intensivstationären Patienten, die aufgrund der Schwere ihrer Erkrankung intensivmedizinisch intubiert werden und in der Regel beatmungspflichtig sind, sondern auch bei normalen Patienten bzw. Personen der Bereich des Mund-Nasen-Rachenraumes räumlich stark begrenzt und sehr empfindlich gegenüber Reinigungsmaßnahmen ist. Insbesondere die Oberfläche der Zunge kann beim Reinigen verletzt werden, sodass eine erhebliche Blutungsgefahr bei der Reinigung der Mundschleimhaut auftritt.

Bei dem bekannten Zungenreiniger ergibt sich das Problem, dass die steifen Profilierungen und die Öffnungen zum Absaugen der angesammelten Anhaftungen direkt, d. h. senkrecht auf die Zungenschleimhaut bzw. senkrecht zur Bewegungsebene oder Bewegungsrichtung des Zungenreinigers gerichtet sind. Demzufolge sind Verletzungen durch die steifen Profilierungen bei der Reinigung und der gleichzeitigen Absaugung möglich. Insbesondere kann durch die senkrecht auf die Zungenschleimhaut gerichteten Öffnungen ein Unterdruck erzeugt werden, sodass sich der Zungenreiniger auf der Zungenschleimhaut festsaugt, wodurch eine zusätzliche Verletzungsgefahr entsteht.

Deshalb liegt der vorliegenden Erfindung die Aufgabe zugrunde, einen Zungenreiniger der eingangs beschriebenen Gattung vorzuschlagen, der eine atraumatische und gleichzeitig leistungsstarke Reinigung und Absaugung ermöglicht.

Diese Aufgabe wird erfindungsgemäß durch die Merkmale des Patentanspruchs 1 gelöst. Weitere vorteilhafte und beanspruchte Weiterbildungen ergeben sich aus den Unteransprüchen und der Beschreibung sowie den Zeichnungen.

Somit wird ein Zungenreiniger zum Reinigen einer Zunge einer Person bzw. eines Patienten, insbesondere eines intensivmedizinisch betreuten Patienten mit zumindest einem Grundkörper und mit einem Griffabschnitt vorgeschlagen, wobei dem Grundkörper zumindest ein Reinigungsbereich zum Reinigen und Absaugen von Anhaftungen auf der Zunge zugeordnet ist. Um eine verletzungssichere und leistungsstarke Reinigung und Absaugung zu realisieren, ist vorgesehen, dass ein erster Reinigungsbereich zumindest eine elastische Reinigungslippe oder dergleichen aufweist, wobei der Reinigungs- bzw. Schablippe zumindest eine Absaugöffnung derart zugeordnet ist, dass die Absaugöffnung im Wesentlichen zumindest parallel zur Bewegungsebene der Reinigungsbewegung des Zungenreinigers ausgerichtet ist.

Auf diese Weise wird ein sicherer und leistungsstarker Aufbau des Zungenreinigers zum Aufbau eines Unterdruckes für die atraumatische Absaugung realisiert. Mit der aus elastischem Material bestehenden Reinigungslippe wird das Sekret bzw. der Zungenbelag sicher und schonend abgelöst und durch die eine oder mehreren Absaugöffnungen vollständig und gleichzeitig schonend abgesaugt, da die Absaugrichtung in Bewegungsrichtung des Zungenreinigers und damit etwa parallel zur Zungenoberfläche bzw. zur Bewegungsebene ausgerichtet ist. Durch die elastische nachgebende Reinigungslippe kann eine unbeabsichtigte Verletzung sicher verhindert werden. Ferner kann ein unbeabsichtigtes Festsaugen des Zungenreinigers an der Zungenoberfläche durch die nicht senkrecht zur Zungenoberfläche sondern zumindest parallel zur Bewegungsebene des Zungenreinigers bzw. zur Zungenoberfläche ausgerichteten Absaugöffnungen ebenfalls sicher verhindert werden.

Weiterhin ist vorgesehen, dass eine Mittelachse zumindest einer Absaugöffnung etwa parallel zur Bewegungsebene der Bewegungsrichtung des Zungenreinigers ausgerichtet und/oder dass eine Mittelachse zumindest einer Absaugöffnung in der Bewegungsebene der Reinigungsbewegung des Zungenreinigers liegt. Dies bedeutet, dass die Mittelachsen bzw. Mittellinien der Absaugöffnungen bzw. -bohrungen etwa parallel zur Zungenoberfläche ausgerichtet sind, die wiederum etwa parallel zur Bewegungsebene ausgerichtet ist. Wenn die Mittelachse zumindest einer Absaugöffnung in der Bewegungsebene liegt, ist dies ein Spezialfall der Parallelität.

Ein besonders konstruktiv einfacher Aufbau des vorgeschlagenen Zungenreinigers ist dadurch realisiert, dass der Grundkörper eine etwa längliche Gehäuseaussparung zum Sammeln der abgelösten Anhaftungen aufweist wobei die Gehäuseaussparung zumindest einen in einem vorbestimmten Radius verlaufenden Randbereichsabschnitt aufweist. Durch das Vorhandensein der in einem Radius bzw. in einer vorbestimmten Krümmung verlaufenden Gehäuseaussparung wird ein Bereich geschaffen, indem die schonend von der elastischen Reinigungslippe abgelösten Anhaftungen optimal gesammelt werden können, um anschließend durch die zugeordneten Absaugöffnungen entfernt zu werden. Es sind auch andere konstruktive Formen möglich, um die atraumatische Absaugung und Reinigung zu realisieren.

Die elastische Reinigungslippe steht etwa senkrecht von einer Vorderseite des Grundkörpers ab und verläuft etwa in dem vorbestimmten Radius des Randbereichsabschnittes der Gehäuseaussparung. Auf diese Weise kann während der Reinigungsbewegung des Zungenreinigers entlang der Bewegungsebene die Reinigungslippe entlang der Zungenfläche bewegt werden und quasi automatisch die abgelösten Anhaftungen innerhalb der Gehäuseaussparung gesammelt werden.

Besonders vorteilhaft ist es hierbei, dass die Reinigungslippe etwa senkrecht an der Vorderseite des Grundkörpers ausgerichtet ist und einen gekrümmten etwa U-förmigen Verlauf quasi parallel zum Randbereichsabschnitt der Gehäuseaussparung aufweist. Demzufolge kann durch die senkrecht ausgerichtete Reinigungslippe eine optimale Reinigungsleistung erzielt werden. Durch den vorzugsweise etwa U-förmigen Verlauf entlang des Randbereichsabschnittes der Gehäuseaussparung werden sämtliche abgelöste Anhaftungen und Sekrete durch die Reinigungslippe gesammelt und gemeinsam der Gehäuseaussparung zugeführt, sodass dort die Absaugung mithilfe eines erzeugten Unterdruckes bzw. eines Vakuums erfolgen kann.

Es sind innere Absaugöffnungen stirnseitig an dem Randbereichsabschnitt der Gehäuseaussparung zum Absaugen der in der Gehäuseaussparung angesammelten Anhaftungen vorgesehen. Durch die stirnseitige Anordnung der Absaugöffnungen wird eine Absaugrichtung parallel zur Bewegungsebene der Reinigungsrichtung bzw. zur Abschabbewegung des Zungenreinigers sichergestellt, um eine Ansaugung an der Zunge sicher zu verhindern. Ferner sind die an dem Randbereichsabschnitt der Gehäuseaussparung angeordneten Absaugöffnungen unmittelbar der Reinigungslippe zugeordnet, wodurch eine optimale Absaugung ermöglicht wird.

Im Rahmen einer nächsten Weiterbildung kann vorgesehen sein, dass äußere Absaugöffnungen oder dergleichen an einem stirnseitigen Randbereich des Grundkörpers vorgesehen sind. Durch diese weiteren äußeren Absaugöffnungen wird eine verbesserte Reinigungswirkung erzielt, da eventuell noch vorhandene abgelöste Anhaftungen nachträglich durch die Absaugöffnung entfernt werden können. Darüber hinaus wird durch diese zusätzliche Absaugöffnung ein Festsaugen selbst bei maximaler Absaugleistung sicher verhindert. Auch diese äußeren Absaugöffnungen sind ebenfalls parallel zur Bewegungsebene des Zungenreinigers ausgerichtet und unmittelbar der Reinigungslippe zugeordnet.

Um die Absaugöffnungen mit einer externen Absaugung zum Erzeugen eines vorbestimmten Unterdruckes konstruktiv einfach verbinden zu können, ist bei dem vorgeschlagenen Zungenreiniger vorgesehen, dass die inneren Absaugöffnungen und die äußeren Absaugöffnungen an einen in dem Grundkörper verlaufenden Absaugkanal oder dergleichen angeschlossen sind, wobei die Absaugöffnungen über den Absaugkanal mit einem Anschluss für eine externe Absaugung zum Erzeugen eines Unterdruckes verbunden sind.

Eine besonders konstruktiv einfache Lösung zum Ansteuern der Absaugleistung bei dem vorgeschlagenen Zungenreiniger wird im Rahmen der Erfindung dadurch realisiert, dass der Griffabschnitt eine von einem Finger des Bedieners abdeckbare Öffnung bzw. einem sogenannten Fingertip aufweist, die bzw. der mit dem Absaugkanal zum Ansteuern der Absaugleistung verbunden ist. Erst durch das Verschließen der Öffnung bzw. des Fingertips oder dergleichen, baut sich ein Unterdruck in dem Absaugkanal bzw. an den Absaugöffnungen auf, wobei der Unterdruck individuell durch die Position des Fingers auf der Öffnung durch den Bediener gesteuert werden oder auch beendet werden kann. Demzufolge wird mit einfachen Mitteln eine optimale und kostengünstige Ansteuerung für eine atraumatische Absaugung realisiert.

Um die Reinigungsleistung bei dem vorgeschlagenen Zungenreiniger weiter zu verbessern, kann beispielsweise vorgesehen sein, dass zum Beispiel an der Rückseite des Grundkörpers ein Reinigungsschwamm oder dergleichen als zweiter Reinigungsbereich vorgesehen ist. Mit dem weichen elastischen Schwamm kann die Zunge vorab gereinigt werden und dabei die Anhaftungen bereits gelöst werden.

Vorzugsweise kann ein Reinigungsschwamm oder auch Reinigungsnoppen oder - bürsten, die entsprechend aus einem weichen, elastischen Material gefertigt sind, eingesetzt werden. Dadurch, dass sich der zweite Reinigungsbereich an der Rückseite des Grundkörpers befindet, kann durch einfaches Drehen des vorgeschlagenen Zungenreinigers die Reinigung mit dem zweiten Reinigungsbereich erfolgen.

Nachfolgend wird die vorliegende Erfindung anhand der Zeichnungen weiter erläutert.

Es zeigen:
- Figur 1: eine schematische Draufsicht auf eine Vorderseite einer möglichen Ausführungsvariante eines erfindungsgemäßen Zungenreinigers;
- Figur 2: eine schematische Draufsicht auf eine Rückseite des Zungenreinigers;
- Figur 3: eine geschnittene Ansicht entlang der Schnittlinie A1-A1 gemäß Figur 1; und
- Figur 4: eine stirnseitige Ansicht auf den Zungenreiniger.

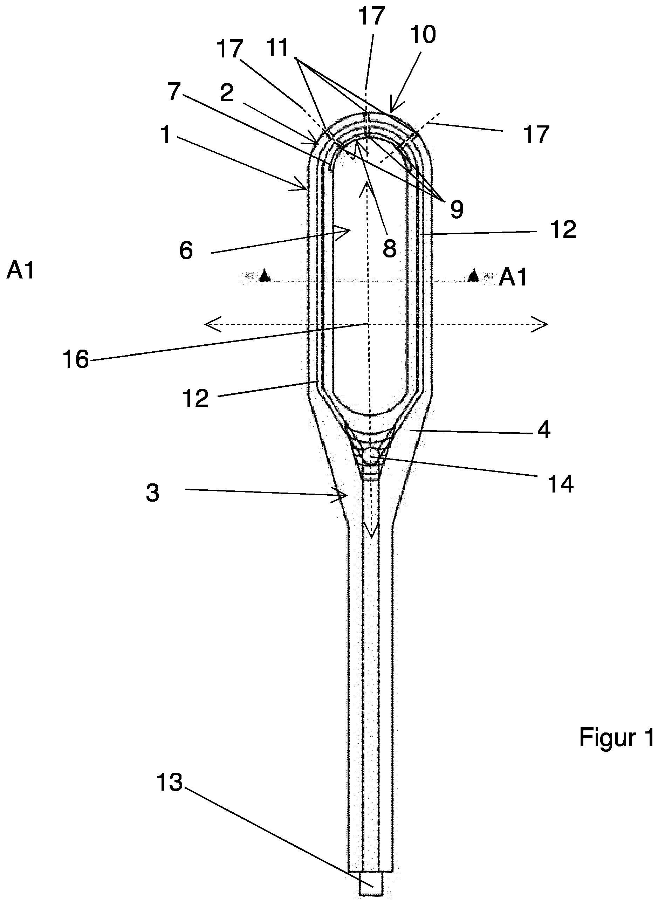

In den Figuren 1 bis 4 sind verschiedene Ansichten eines erfindungsgemäßen Zungenreinigers 1 zum Reinigen einer Zunge einer Person, insbesondere eines intensivmedizinisch betreuten Patienten lediglich beispielhaft dargestellt.

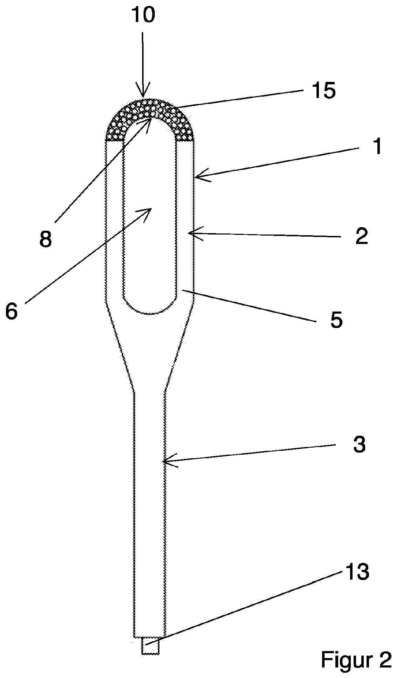

Der Zungenreiniger 1 umfasst einen Grundkörper 2 und einen Griffabschnitt 3 zum Handhaben durch einen Bediener. Der Grundkörper 2 weist eine Vorderseite 4 mit einem ersten Reinigungsbereich und eine Rückseite 5 mit einem zweiten Reinigungsbereich auf. Der Grundkörper 2 weist eine längliche Gehäuseaussparung 6 zum Sammeln der abgelösten Anhaftungen auf, die quasi in dem Grundkörper 2 eine durchgehende Öffnung bildet. Wie insbesondere aus den Figuren 1 und 2 ersichtlich ist, weist die längliche Gehäuseaussparung 6 jeweils an ihren Stirnseiten einen in einem vorbestimmten Radius verlaufenden Randbereichsabschnitt 8 auf.

Der an der Vorderseite des Grundkörpers 2 vorgesehene erste Reinigungsbereich weist eine elastische Reinigungslippe 7 auf. Die Reinigungslippe 7 verläuft an der Vorderseite des Grundkörpers 2 entlang des gekrümmten Randbereichsabschnittes 8 der Gehäuseaussparung 6. Die Reinigungslippe 7 ist etwa senkrecht an der Vorderseite des Grundkörpers 2 ausgerichtet und weist einen gekrümmten etwa U-förmigen Verlauf parallel zum Randbereichsabschnitt 8 der Gehäuseaussparung 6 auf.

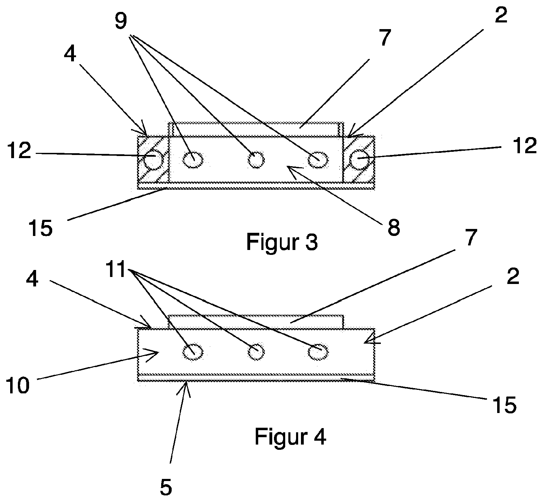

Demzufolge bildet der Randbereichsabschnitt 8 der Gehäuseaussparung 6 eine innere Kurvatur, an der mehrere innere Absaugöffnungen 9 stirnseitig zum Absaugen der innerhalb der Gehäuseaussparung 6 angesammelten Anhaftungen angeordnet sind. Wie insbesondere aus Figur 3 ersichtlich ist, sind die ersten inneren Absaugöffnungen 9 symmetrisch nebeneinander entlang des gekrümmten Randbereichsabschnittes 8 der Gehäuseaussparung angeordnet.

An einem dem gekrümmten Randbereichsabschnitt 8 der Gehäuseaussparung 6 gegenüberliegenden stirnseitigen Randbereich 10 des Grundkörpers 2 sind äußere Absaugöffnungen 11 vorgesehen, wie dies beispielsweise aus Figur 4 deutlich wird. Der stirnseitige Randbereich 10 des Grundkörpers 2 bildet quasi eine äußere Kurvatur des Grundkörpers 2 bei dem vorgeschlagenen Zungenreiniger 1. Auch die äußeren Absaugöffnungen 11 sind entlang des stirnseitigen Randbereiches 10 symmetrisch nebeneinander angeordnet.

Gemäß Figur 1 sind die inneren Absaugöffnungen 9 an der inneren Kurvatur bzw.an dem Randbereichsabschnitt 8 der Gehäuseaussparung 6 an dem Grundkörper 2 und die äußeren Absaugöffnungen 11 an der äußeren Kurvatur bzw. an dem stirnseitigen Randbereich 10 des Grundkörpers 2 der Reinigungslippe 7 unmittelbar zugeordnet, da die Reinigungslippe 7 zwischen dem Randbereichsabschnitt 8 der Gehäuseaussparung 6 und dem stirnseitigen Randbereich 10 an dem Grundkörper 2 angeordnet ist.

Ferner ist in Figur 1 die Bewegungsebene 16 der Bewegungsrichtung des Zungenreinigers 1 zum Reinigen anhand von kreuzförmig zu einander angeordneten gestrichelten Doppelpfeilen angedeutet. Die Doppelpfeile spannen quasi die Bewegungsebene 16 in der Zeichnungsebene auf. Zum Absaugen der auf der Zunge mit der elastischen Reinigungslippe 7 abgelösten Anhaftungen sind die inneren Absaugöffnungen 9 und die äußeren Absaugöffnungen 11 etwa parallel zur Bewegungsebene 16 der Reinigungsrichtung oder Abschabrichtung des Zungenreinigers 1 ausgerichtet. Demzufolge sind die als gestrichelten Linien angedeuteten Mittelachsen 17 der Absaugöffnungen 9, 11 parallel zur Bewegungsebene 16 ausgerichtet. Da sich die inneren Absaugöffnungen 9 und die äußeren Absaugöffnungen 11 an dem gekrümmten Randbereichsabschnitt 8 bzw. an dem gekrümmten stirnseitigen Randbereich 10 des Grundkörpers 2 befinden, sind die Mittelachsen 17 der Absaugöffnungen 9, 11 in einem vorbestimmten Winkel zueinander ausgerichtet. Ungeachtet dessen sind die Mittelachsen 17 entweder parallel zur Bewegungsebene 16 ausgerichtet oder liegen in der Bewegungsebene 16 des Zungenreinigers 1.

Zur Absaugung der Anhaftungen sind die inneren Absaugöffnungen 9 und die äußeren Absaugöffnungen 11 an einen Absaugkanal 12 angeschlossen, die in dem Grundkörper 2 des Zungenreinigers 1 verlaufen. Über den Absaugkanal 12 werden die Absaugöffnungen 9, 11 mit einem Anschluss 13 für eine nicht weiter dargestellte externe Absaugung verbunden. Der Absaugkanal 12 ist in Figur 1 mit gestrichelter Linie im Inneren des Grundkörpers 2 des Zungenreinigers 1 angedeutet.

Zum Ansteuern der Absaugleistung ist an dem Griffabschnitt 3 des Zungenreinigers 1 eine von einem Finger des Bedieners abdeckbare Öffnung 14 vorgesehen, die mit dem Absaugkanal 12 zum Ansteuern der Absaugleistung verbunden ist. Vorzugsweise ist die Öffnung 14 auf der Vorderseite des Grundkörpers 2 wie der erste Reinigungsbereich mit der Reinigungslippe 7 an dem Griffabschnitt 3 vorgesehen, sodass der Bediener ergonomisch einfach die Öffnung 14 mit seinem Zeigefinger bedienen bzw. abdecken kann.

Wie insbesondere aus Figur 2 deutlich wird, ist auf der Rückseite des Grundkörpers 2 des vorgeschlagenen Zungenreinigers 1 ein zweiter Reinigungsbereich mit einem Reinigungsschwamm 15 vorgesehen. Mit dem aus weichem elastischem Material bestehenden Reinigungsschwamm 15 kann der Bediener zunächst die Anhaftungen auf der Zunge des Patienten lösen, um anschließend mit der Reinigungslippe 7 diese Anhaftungen von der Zunge durch die Absaugöffnungen 9, 11 zu entfernen.

### Bezugszeichen

- 1: Zungenreiniger
- 2: Grundkörper
- 3: Griffabschnitt
- 4: Vorderseite des Grundkörpers
- 5: Rückseite des Grundkörpers
- 6: Gehäuseaussparung
- 7: elastische Reinigungslippe
- 8: Randbereichsabschnitt der Gehäuseaussparung
- 9: innere Absaugöffnung
- 10: stirnseitiger Randbereich des Grundkörpers
- 11: äußere Absaugöffnung
- 12: Absaugkanal
- 13: Anschluss für eine externe Absaugung
- 14: Öffnung zum Steuern der Absaugleistung
- 15: Reinigungsschwamm
- 16: Bewegungsebene zum Reinigen
- 17: Mittelachse der Absaugöffnung

## Patentansprüche

1. Zungenreiniger (1) zum Reinigen einer Zunge eines Patienten mit zumindest einem Grundkörper (2) und mit einem Griffabschnitt (3), wobei dem Grundkörper (2) zumindest ein Reinigungsbereich zum Reinigen und Absaugen von Anhaftungen auf der Zunge zugeordnet ist, wobei ein erster Reinigungsbereich zumindest eine elastische Reinigungslippe (7) aufweist, wobei der Reinigungslippe (7) zumindest eine Absaugöffnung (9, 11) derart zugeordnet ist, dass eine Mittelachse (17) zumindest einer Absaugöffnung (9, 11) etwa parallel zur Bewegungsebene (16) der Reinigungsbewegung des Zungenreinigers (1) ausgerichtet ist oder eine Mittelachse (17) zumindest einer Absaugöffnung (9, 11) in der Bewegungsebene (16) der Reinigungsbewegung des Zungenreinigers (1) liegt, wobei der Grundkörper (2) eine längliche Gehäuseaussparung (6) zum Sammeln der abgelösten Anhaftungen aufweist, wobei die Gehäuseaussparung (6) zumindest einen in einem vorbestimmten Radius verlaufenden Randbereichsabschnitt (8) aufweist, wobei die Reinigungslippe (7) etwa senkrecht von einer Vorderseite des Grundkörpers (2) absteht und etwa in dem vorbestimmten Radius des Randbereichsabschnittes (8) der Gehäuseaussparung (6) verläuft, und
**dadurch gekennzeichnet, dass**
innere Absaugöffnungen (9) stirnseitig an dem Randbereichsabschnitt (8) der Gehäuseaussparung (6) zum Absaugen der in der Gehäuseaussparung (6) angesammelten Anhaftungen vorgesehen sind.

2. Zungenreiniger (1) nach Anspruch 1, **dadurch gekennzeichnet, dass** äußere Absaugöffnungen (11) an einem stirnseitigen Randbereich (10) des Grundkörpers (2) vorgesehen sind.

3. Zungenreiniger (1) nach Anspruch 2, **dadurch gekennzeichnet, dass**
die inneren Absaugöffnungen (9) und die äußeren Absaugöffnungen (11) an einen in dem Grundkörper (2) verlaufenden Absaugkanal (12) angeschlossen sind, wobei die Absaugöffnungen (9, 11) über den Absaugkanal (12) mit einem Anschluss (13) für eine externe Absaugung verbunden sind.

4. Zungenreiniger (1) nach einem der vorangehenden Ansprüche, **dadurch gekennzeichnet, dass** der Griffabschnitt (3) eine von einem Finger des Bedieners abdeckbare Öffnung (14) zum Ansteuern der Absaugleistung aufweist.

5. Zungenreiniger (1) nach Anspruch 4, **dadurch gekennzeichnet, dass** die abdeckbare Öffnung (14) mit dem Absaugkanal (12) verbunden ist.

6. Zungenreiniger (1) nach einem der vorangehenden Ansprüche, **dadurch gekennzeichnet, dass** an einer Rückseite des Grundkörpers (2) ein Reinigungsschwamm (15) als zweiter Reinigungsbereich vorgesehen ist.

## Claims

1. A tongue cleaner (1) for cleaning the tongue of a patient, having at least one main part (2) and having a handle section (3), wherein the main part (2) is paired with at least one cleaning region for cleaning and suctioning deposits on the tongue, wherein a first cleaning region has at least one elastic cleaning lip (7), said cleaning lip (7) being paired with at least one suction opening (9, 11) such that a central axis (17) of at least one suction opening (9, 11) is aligned approximately in parallel to the movement plane (16) of the cleaning movement of the tongue cleaner (1) or a central axis (17) of at least one suction opening (9, 11) is in the movement plane (16) of the cleaning movement of the tongue cleaner (1), wherein the main part (2) has an oblong housing recess (6) for collecting the detached deposits, wherein the housing recess (6) has at least one peripheral section (8) extending at a predetermined radius, wherein the cleaning lip (7) is approximately orthogonally spaced apart from a front side of the main part (2) and extends approximately at the predetermined radius of the peripheral section (8) of the housing recess (6), and **characterised in that** inner suction openings (9) are provided frontally on the peripheral section (8) of the housing recess (6) for suctioning the deposits accumulated in the housing recess (6).

2. The tongue cleaner (1) according to claim 1, **characterised in that** outer suction openings (11) are provided at a frontal periphery (10) of the main part (2).

3. The tongue cleaner (1) according to claim 2, **characterised in that** the inner suction openings (9) and the outer suction openings (11) are coupled to a suction duct (12) extending in the main part (2), wherein the suction openings (9, 11) are connected to a port (13) via the suction duct (12) for external suction.

4. The tongue cleaner (1) according to any one of the preceding claims, **characterised in that** the handle section (3) has an opening (14) coverable by a finger of the operator for controlling the suction power.

5. The tongue cleaner (1) according to claim 4, **characterised in that** the coverable opening (14) is connected to the suction duct (12).

6. The tongue cleaner (1) according to any one of the preceding claims, **characterised in that** a cleaning sponge (15) is provided on a rear side of the main part (2) as a second cleaning region.

## Revendications

1. Nettoyeur de langue (1) destiné à nettoyer une langue d'un patient, présentant au moins un corps de base (2) et une partie manche (3), au moins une zone de nettoyage destinée à nettoyer et à aspirer les dépôts recouvrant la langue étant affectée au corps de base (2), une première zone de nettoyage présentant au moins une lèvre de nettoyage (7) élastique, au moins une ouverture d'aspiration (9, 11) étant affectée à la lèvre de nettoyage (7) de telle sorte qu'un axe central (17) d'au moins une ouverture d'aspiration (9, 11) est orienté de manière sensiblement parallèle au plan de mouvement (16) dans lequel s'effectue le mouvement de nettoyage du nettoyeur de langue (1) ou qu'un axe central (17) d'au moins une ouverture d'aspiration (9, 11) se situe dans le plan de mouvement (16) dans lequel s'effectue le mouvement de nettoyage du nettoyeur de langue (1), le corps de base (2) présentant un évidement de boîtier (6) de forme allongée destiné à recueillir les dépôts détachés, l'évidement de boîtier (6) présentant au moins une zone de partie périphérique (8) s'étendant selon un rayon prédéterminé, la lèvre de nettoyage (7) dépassant de manière sensiblement perpendiculaire d'une face avant du corps de base (2) et s'étendant sensiblement selon le rayon prédéterminé de la zone de partie périphérique (8) de l'évidement de boîtier (6), et **caractérisé en ce que** des ouvertures d'aspiration intérieures (9) sont prévues, côté frontal, sur la zone de partie périphérique (8) de l'évidement de boîtier (6) dans le but d'aspirer les dépôts recueillis dans l'évidement de boîtier (6).

2. Nettoyeur de langue (1) selon la revendication 1, **caractérisé en ce que** des ouvertures d'aspiration extérieures (11) sont prévues sur une partie périphérique frontale (10) du corps de base (2).

3. Nettoyeur de langue (1) selon la revendication 2, **caractérisé en ce que** les ouvertures d'aspiration intérieures (9) et les ouvertures d'aspiration extérieures (11) sont raccordées à un canal d'aspiration (12) s'étendant dans le corps de base (2), les ouvertures d'aspiration (9, 11) étant reliées par l'intermédiaire du canal d'aspiration (12) à un raccord (13) prévu pour un dispositif d'aspiration externe.

4. Nettoyeur de langue (1) selon l'une quelconque des revendications précédentes, **caractérisé en ce que** la partie manche (3) présente une ouverture (14) qui peut être recouverte par un doigt de l'utilisateur en vue de commander la puissance d'aspiration.

5. Nettoyeur de langue (1) selon la revendication 4, **caractérisé en ce que** l'ouverture (14) recouvrable est reliée au canal d'aspiration (12).

6. Nettoyeur de langue (1) selon l'une quelconque des revendications précédentes, **caractérisé en ce qu'**une éponge de nettoyage (15) est prévue en tant que seconde zone de nettoyage sur la face arrière du corps de base (2).
